# EUROPEAN PATENT APPLICATION

(11) **EP 2 602 240 A1**
(43) Date of publication of application: **12.06.2013**
(21) Application number: 11192631.7
(22) Date of filing: 08.12.2011
(51) Int. Cl.: C07C 51/47, C02F 1/28

(54) **A process for extraction of aromatic dicarboxylic acids**

(71) Applicant: SOLVAY SA, 1120 Bruxelles (BE)
(72) Inventor: Dournel, Pierre, 1030 Brussels (BE)
(74) Representative: Vande Gucht, Anne

(57) **Abstract**

The present invention relates to a process for extraction of aromatic dicarboxylic acids of Formula I from an aqueous solution with a non-ionic polystyrene resin. The process of the present invention can be employed for the treatment of aqueous solutions such as industrial waste water or leachate resulting from household or industrial waste.

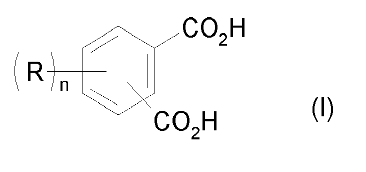

## Description

The present invention relates to a process for extraction of aromatic dicarboxylic acids from an aqueous solution. The process of the present invention can be employed for the treatment of aqueous solutions such as industrial waste water or leachate resulting from household or industrial waste.

Aromatic dicaboxylic acids are common degradation products present in waste water streams of various industrial plants. These compounds usually show only a poor biodegradability and therefore need to be removed from the main waste water stream before a biological waste water treatment can take place.

In addition, aromatic dicarboxylic acids are commonly used as intermediates for the manufacturing of diverse chemical products. Thus, aromatic dicarboxylic acids recovered from the waste water can be directly used for these purposes if their purity is sufficiently high.

In order to enable a direct isolation of aromatic dicarboxylic acids in a sufficiently high purity, the corresponding process for their extraction needs to be sufficiently selective and allow an efficient separation of these compounds from other impurities present in the waste water stream.

Use of synthetic resins for isolation of organic acids from aqueous solutions is known in the prior art, whereby non-ionic resins as well as ion-exchange resins are used for this purpose. Such processes can also be employed for the isolation of aromatic carboxylic acids.

I. do Nascimento Filho et al. (Journal of Chromatography A, 1027, 2004, pages 167-170) describe a process for selective extraction of benzoic acid from landfill leachate samples. The samples were submitted to solid-phase extractions with XAD-4 resin as the stationary phase and ion-exchange chromatography using the ion-exchange resin Amberlyst® A-27. The pH of the samples varied from 7 to 8 and under these conditions Amberlyst® A-27 resin showed to be highly selective for benzoic acid extractions. However, no solid-phase extractions of aromatic dicarboxylic acids are reported in this study.

G. R. Aiken (Advances in Chemistry, 1986, pages 295-307) describes isolation of organic acids from large volumes of water by adsorption on non-ionic resins Amberlite® XAD-8 and XAD-4 and on anion-exchange resin Duolite® A-7. The eluates were adjusted to pH 2. XAD-8, an acrylic ester resin, was shown to absorb a variety aromatic and aliphatic carboxylic acids. However, no noticeable selectivity which would allow a selective extraction of aromatic dicarboxylic acids from the aqueous solution was observed.

Thus, there is a significant demand for an efficient and selective extraction method of aromatic dicarboxylic acids from an aqueous solution. Such extraction method should be applicable for the treatment of a wide variety of industrial waste water streams containing other water-soluble organic compounds such as aliphatic and aromatic carboxylic acids, alcohols, phenols and salts of amines. Moreover, the desired method should allow a selective extraction of halogenated and/or alkylated aromatic dicarboxylic acids in the presence of the corresponding unsubstituted aromatic dicarboxylic acids.

The above technical problem has been solved by the present invention. The authors of the present invention surprisingly found that a broad range of structurally diverse aromatic dicarboxylic acids can be efficiently and selectively extracted from the aqueous solution by using a non-ionic polystyrene resin.

Thus, the present invention relates to a process for extraction of an aromatic dicarboxylic acid of Formula I 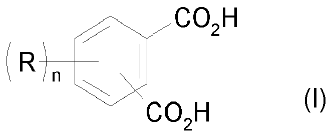 wherein
R is independently selected from group consisting of halogen, C₁₋₁₂ alkyl and halogenated C₁₋₁₂ alkyl,
n is 1, 2 or 3,
from an acidic aqueous solution,
wherein the process comprises contacting the aqueous solution with a non-ionic polystyrene resin.

In the process of the present invention, the aqueous solution containing the aromatic dicarboxylic acid of Formula I is brought into contact with a non-ionic polystyrene resin. For this purpose the aqueous solution may be mixed with the non-ionic polystyrene resin in a bed process, or preferably put through a column filled with the non-ionic polystyrene resin. The flow rate of the aqueous solution through the resin-filled column is adjusted depending on factors, such as for instance concentration of the aromatic dicarboxylic acid in the aqueous solution or desired residual content of the aromatic dicarboxylic acid in the effluent leaving the column. Preferably, the residence time of the aqueous solution in the column is between 5 and 20 minutes, more preferably between 6 and 15 minutes and particularly preferred between 8 and 12 minutes.

The process for extraction typically allows removal of at least 60 wt.-%, preferably at least 70 wt.-%, even more preferred at least 80 wt.-% and particularly preferred at least 90 wt.-% of the aromatic dicarboxylic acid from the aqueous solution.

The total amount of the non-ionic polystyrene resin used for the process for extraction can be adjusted depending on the desired extraction selectivity and the residual content of the aromatic dicarboxylic acid of Formula I in the effluent leaving the column. Thus, the volume of the aqueous solution is typically between 20 m³ to 200 m³, particularly preferred between 45 m³ and 150 m³ per ton of the non-ionic polystyrene resin employed.

The process for extraction can be carried out at a temperature between 0°C and 70°C, preferably between 10°C and 50°C, particularly preferred between 20°C and 30°C.

The process for extraction is preferably carried out at atmospheric pressure. However, it is possible to carry out the process for extraction at a pressure between 10.0 kPa and 101.3 kPa or at a pressure which is higher than 101.3 kPa. Hereinafter the term "atmospheric pressure" refers to a pressure of 101.3 kPa.

The process for extraction can be selectively carried out in the presence of water-soluble organic compounds such as alcohols, ketones, phenols, aliphatic and aromatic carboxylic acids and salts of aliphatic and aromatic amines. Moreover, the process for extraction can be performed in the presence of inorganic cations such as sodium, potassium, magnesium and/or inorganic anions such as sulfate, nitrate and phosphate.

In accordance with the present application the process for extraction is carried out "selectively" if the content of the desired aromatic dicarboxylic acid reaches at least temporarily more than 70 wt.-%, preferably more than 80 wt.-% of all organic compounds absorbed on the non-ionic polystyrene resin during the extraction.

The effluent from the extraction column can be subsequently subjected to a biological waste water treatment, to an additional solid-phase process for extraction, to any other extraction process or to any other suitable process known in the prior art.

According to the present invention, the non-ionic polystyrene resin used in the process for extraction has no ionic functional groups and is therefore a completely non-ionic hydrophobic polymer. Without wishing to be bound by any theory, it is believed that an interaction between the phenyl moieties of the non-ionic polystyrene resin and the aromatic dicarboxylic acid is responsible for an efficient and selective extraction of the aromatic dicarboxylic acid of Formula I from the aqueous solution. Importantly, during the process for extraction the absorption of other aromatic compounds resulting from oxidation of aromatic dicarboxylic acids and having alcoholic or phenolic groups is significantly lower than the absorption of aromatic dicarboxylic acids of Formula I. Moreover, the process for extraction allows a selective separation of halogenated and/or alkylated aromatic dicarboxylic acids from unsubstituted aromatic dicarboxylic acids.

The non-ionic polystyrene resin used in the process of the present invention is not particularly limited and therefore any commercially available non-ionic polystyrene resin can be employed.

In a preferred embodiment of the present invention the non-ionic polystyrene resin is a *para*-divinylbenzene cross-linked polystyrene resin. This resin is for instance obtainable by suspension polymerisation of styrene with a content of about 5 to 15 wt.-%, especially about 8 to 12 wt.-% of divinyl benzene.

The non-ionic polystyrene resin used in the process for extraction has a sufficiently large specific surface area to enable a sufficiently fast and efficient adsorption of aromatic dicarboxylic acid of Formula I. Preferably, the specific surface area of the non-ionic polystyrene resin is higher than 400 m²/g, preferably higher than 500 m²/g, even more preferred higher than 600 m²/g and particularly preferred higher then 700 m²/g.

In a preferred embodiment of the present invention the pore volume of the non-ionic polystyrene resin is chosen to be higher than 0.2 ml/ml, even more preferred higher than 0.35 ml/ml and particularly preferred higher than 0.45 ml/ml.

In a particularly preferred embodiment of the present invention, the non-ionic polystyrene resin is a para-divinylbenzene cross-linked polystyrene resin having a specific surface area of not less than 750 m²/g,
a pore volume of not less than 0.5 ml/ml,
a particle size between 0.3 mm and 1.2 mm and
a pore envelope between 5.5 nm and 8.0 nm.

The corresponding non-ionic polystyrene resin is manufactured by Rohm and Haas Company and is commercially available under the tradename Amberlite® XAD-4.

The process for extraction is carried out under aqueous acidic conditions. Thus, the aqueous solution employed in the process for extraction of the present invention has a pH value below 7.0, preferably below 6.0, still more preferred below 5.0, even more prefered below 4.0, yet even more preferred below 3.0, more preferred below 2.0 and particularly preferred below 1.5.

Thus, the preferable pH value of the aqueous solution ranges from 7.0 to - 0.5, more preferred from 5.0 to -0.1, even more preferred from 3.0 to 0 and particularly preferred from 2.0 to 0.5.

Under these conditions, at least 90 wt.-%, preferably at least 95 wt.-% of the aromatic dicarboxylic acids of Formula I in the aqueous solution are present in their non-ionized form. Under these conditions the process for extraction is particularly efficient and selective.

The aromatic dicarboxylic acid of Formula I can be a derivative of phthalic, isophthalic or terephthalic acid. Preferably, the aromatic dicarboxylic acid of Formula I is a derivative of phthalic acid.

In the Formula I the halogen atom is selected from the group consisting of a fluorine atom, a chlorine atom, a bromine atom and an iodine atom. In a preferred embodiment of the present invention the halogen atom is a chlorine atom.

The C₁₋₁₂ alkyl group may be linear or branched, and examples thereof include methyl, ethyl, *n*-propyl, *iso*-propyl, *n*-butyl, *iso*-butyl, *sec*-butyl, *tert-*butyl, *n*-amyl, *sec*-amyl, *tert*-amyl, *n*-hexyl and *n*-heptyl. Preferably, R is selected from the group consisting of *n*-amyl, *sec*-amyl and *tert*-amyl, whereby *sec*-amyl and *tert*-amyl are particularly preferred.

The halogenated C₁₋₁₂ alkyl group is a C₁₋₁₂ alkyl group as defined above, which is substituted by at least 1 halogen atom.

In the Formula I n is preferably 1.

In one of the preferred embodiments of the present invention the aromatic dicarboxylic acid of Formula I is a 4-alkylphthalic acid, particularly preferred a 4-amylphthalic acid. In a particularly preferred embodiment the aromatic dicarboxylic acid of Formula I is selected from the group consisting of *4-tert-*amylphthalic acid and 4-sec-amylphthalic acid.

In the text of the present application the term "aromatic dicarboxylic acid" can apply to a single compound or to a mixture of isomers.

Another aspect of the present invention relates to a process for removal of the aromatic dicarboxylic acid of Formula I from an aqueous solution, wherein the process comprises the following steps:
a) adjusting the pH value of the aqueous solution to a value below 7.0;
b) extracting the composition obtained in step a) with a water non-miscible solvent;
c) extracting the aqueous solution obtained in step b) according to the present invention; and
d) extracting the aromatic dicarboxylic acid of Formula I from the non-ionic polystyrene resin with a water miscible solvent, wherein a solution of the aromatic dicarboxylic acid of Formula I is obtained.

In a preferred embodiment of the present invention the pH value of the aqueous solution in step a) is adjusted by addition of an acid, which is optionally diluted with water. The choice of the acid which can be used for the adjustment of the pH value of the aqueous solution in step a) is not particularly limited as long as the acid has a sufficient strength for achieving the desired pH value, does not interfere with the process for removal of the aromatic dicarboxylic acid from the aqueous solution and does not cause the decomposition of the aromatic dicarboxylic acid. For example, Br⌀nsted acids such as sulfuric acid, hydrochloric acid, phosphoric acid, or nitric acid can be employed for this purpose. Alternatively, the pH value of the aqueous solution can be adjusted upon addition of a salt such as sodium hydrogen sulfate or of an aqueous solution of this salt.

The pH value of the aqueous solution can be determined by any appropriate test method known in the prior art. The measurement is preferably carried out using a glass electrode as a sensor at a temperature of 25±0.1°C. For pH values ranging from 0.5 to 14 the standard test method ASTM D 1293-99 (reapproved 2005), test method B can be employed. pH meters and glass electrodes suitable for this purpose are known in the prior art and are obtainable e.g. from Metrohm AG (Herisau, Switzerland). An example of a suitable pH meter is Metrohm 827 pH Lab, equipped with a combined pH glass electrode, filled with 3 mol/l KCl solution such as, for instance, Unitrode.

When pH values below 0.5 are obtained using the standard test method ASTM D 1293-99 (reapproved 2005), or when the target pH value to be adjusted is below 0.5, then the pH should be measured by using the Pitzer method as outlined by D. K. Nordstrom et al. (Environ. Sci. Technol. 34, 2000, pages 254-258), whereby an Orion Ross combination glass electrode with a KCl filling solution can be employed as a sensor. The corresponding glass electrode, such as for instance, PerpHecT ROSS, is commercially available, *inter alia* from Thermo Electron Corporation (Marietta, Ohio, USA).

The adjustment of the pH value in step a) can be accompanied by a precipitation of crystalline or amorphous compounds. Therefore, the composition obtained in step a) is consequently extracted with a water non-miscible solvent whereby the precipitated compounds are at least partially dissolved. Preferably, the water non-miscible solvent employed in step b) is a low polarity organic solvent having a boiling point/distillation range between 50 and 250°C at atmospheric pressure. Boiling points and distillation ranges of organic solvents can be determined by the test method ASTM D 1078-05. Distillation ranges of petroleum products can be further determined by the test method ASTM D 86-11a.

The choice of the water non-miscible solvent is not particularly limited as long as it is suitable for the liquid-liquid extraction of the composition obtained in step a). For instance, solvents such as toluene, xylene, n-heptane, diisobutylcarbinol (DBC), a mixture of aromatic hydrocarbons or light petroleum can be used for this purpose. The particularly preferred water non-miscible solvent employed in step b) is a mixture of aromatic hydrocarbons having a distillation range of about 181 to about 208°C at atmospheric pressure and an aromatic content of more than 98 wt.-%, preferably more than 99 wt.-%. This mixture is commercially avilable from ExxonMobil Chemical under the trade name Solvesso™ 150. In an alternative embodiment of the present invention, a mixture of Solvesso™ 150 and DBC can be used in step b).

Step c) of the process for removal of the aromatic dicarboxylic acid of Formula I from an aqueous solution is the process for extraction according to the present invention.

In step d), the aromatic dicarboxylic acid is extracted from the non-ionic polystyrene resin with a water-miscible solvent, whereby a solution of the aromatic dicarboxylic acid is obtained and the non-ionic polystyrene resin is regenerated for use in step c). The water-miscible solvent used for this purpose is fully miscible with water and is preferably biodegradable. Preferably, the water-miscible solvent used for this purpose is an organic solvent having a boiling point/distillation range between 30 and 100°C, particularly preferred between 50 and 80°C at atmospheric pressure. In a particularly preferred embodiment of the present invention, the water-miscible solvent is selected from the group consisting of methanol, ethanol and acetone, whereby methanol is particularly preferred.

In one of the embodiments of the present invention, the process for removal of the aromatic dicarboxylic acid from an aqueous solution is used for recovery of the aromatic dicarboxylic acid. In this embodiment the solution of the aromatic dicarboxylic acid obtained in step d) above is subsequently evaporated to yield the aromatic dicarboxylic acid having a purity above 80 wt.-%, preferably in a purity close to 85 wt.-%.

In this embodiment, the water non-miscible solvent used in step b) is preferably an organic solvent having a low polarity, for instance light petroleum. The aromatic dicarboxylic acid has only a limited solubility in the water non-miscible solvent, whereby the insoluble precipitates formed in step a) are dissolved. Thus, the recovery yield of the aromatic dicarboxylic acid from the aqueous solution is maximized.

In this embodiment, one ton of the non-ionic polystyrene resin is employed for the treatment of between 100 m³ and 200 m³, for instance 150 m³ of the aqueous solution. Under these conditions the adsorption capacity of the non-ionic polystyrene resin is about 90 g of aromatic dicarboxylic acid / kg of resin.

Under these conditions, the effluent after the column typically contains between 100 mg/l and 300 mg/l, for instance about 200 mg/l of non-biodegradable refractory aromatic compounds. Therefore, the effluent after the column is not biodegradable and needs to be objected to an additional treatment, such as a second column or a UV/H₂O₂ treatment.

In another embodiment of the present invention the process for removal of an aromatic dicarboxylic acid directly precedes the biological treatment of the aqueous solution. In this embodiment, the volume of the aqueous solution that is subjected to the process of the present invention is limited to about 45 m³ per tonne of the non-ionic polystyrene resin. In this embodiment the water-miscible solvent needs to be a biodegradable organic solvent, because it will be present in the effluent after the column and will be partially sent to the bioplant after the column has been regenerated as specified in step d) above. The solution of the aromatic dicarboxylic acid obtained in step d) can be incinerated together with the solution containing the water non-miscible solvent, which was obtained in step b).

Preferably, the aqueous solution treated by the processes of the present invention is industrial waste water.

In a particularly preferred embodiment of the present invention the aromatic dicarboxylic acid of Formula I is selected from the group consisting of 4-tert-amylphthalic acid and 4-sec-amylphthalic acid. If both of these compounds are present in the aqueous solution, a mixture of both compounds is isolated.

Amylphthalic acids are commercially not available and can, for instance, be used as starting materials for the preparation of a broad range of chemical products.

### Description of the drawings

Figure 1 shows the evolution of total organic carbon (TOC) in the effluent after the column containing Amberlite® XAD-4 and having a pH of 1.1.
Figure 2 shows evolution of the concentration of various components after passing through the column containing Amberlite® XAD-4 at pH 1.1.
Figure 3 illustrates evolution of main aliphatic acids during adsorption on a column containing Amberlite® XAD-4 at pH 1.1.
Figure 4 illustrates avolution of minor aliphatic acids during adsorption on a column containing Amberlite® XAD-4 at pH 1.1.
Figure 5 shows adsorption selectivity of phthalic acid, 4-*tert*-amylphthalic acid, 4-sec-amylphthalic acid, amylphthalic acid derivatives and other aromatic compounds by Amberlite® XAD-4 at pH 13.
Figure 6 shows normalized compositions of the aromatic fraction before and after adsorption on Amberlite® XAD-4 at pH 1.1.

### Examples

The following non-limiting examples will illustrate representative embodiments of the invention in detail.

In the following examples, 45 g of Amberlite® XAD-4 purchased from Sigma-Aldrich were used as such (the commercial product contains a significant amount of water). The resin was used to fill a column. The aqueous solution was passed through the column at a constant flow rate (typically 5 ml/min) which corresponds to a residence time of about 10 minutes. All experiments were carried out at a temperature of 25°C and at atmospheric pressure.

The effluent after the column was regularly collected and analyzed. If not specified otherwise, the amounts of components detected are given in mg/l as determined by high performance liquid chromatography (HPLC).

### 1. Example 1 - Tests performed at low pH

The following extraction tests were carried out using an acidic aqueous solution. A sample of industrial waste main streams of a Solvay production unit was collected.

This effluent sample was acidified to pH 1.0 upon addition of 96 wt.-% sulfuric acid and extracted with a mixture of Solvesso™ 150 and diisobutylcarbinol (DBC), (vol.-ratio effluent : solvent of 10 : 1).

The resulting aqueous solution was characterized by a low pH (1.1) and a TOC value of 1750 mg/l. It was used as such without any pretreatment.

A total volume of 10 litres was passed through a column containing 45 g of Amberlite® XAD-4 and samples of the effluent after the column were regularly collected for analysis. The flow rate was adjusted to a constant value of 6 ml/min using a peristaltic pump, so that the total residence time in the column was 12 minutes.

### TOC evolution

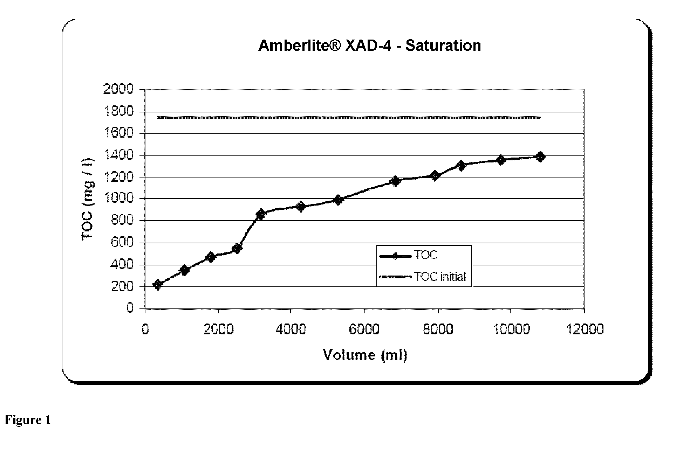

The evolution of TOC values in the effluent after the column is shown in Figure 1.

This curve shows the existence of a dynamic equilibrium inside the column. At the beginning most of the compounds are adsorbed (low selectivity), then these components are progressively displaced and desorbed to be replaced by others. After 10 litres of effluent, the TOC value stabilizes at a value close to 1400 mg/l which corresponds to 80 % of the initial value.

### 1.2 Detailed analysis

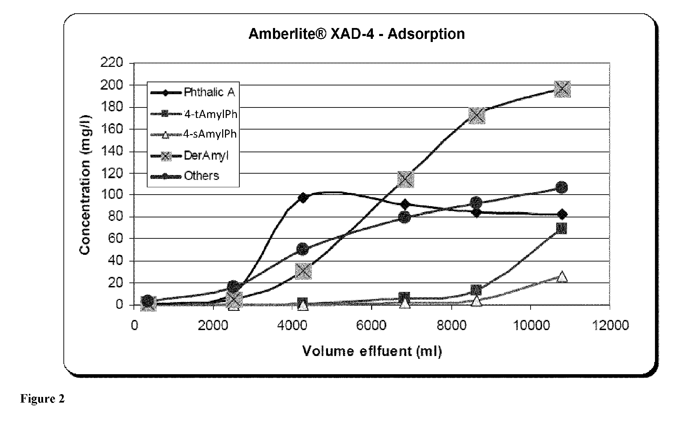

The collected samples were analyzed by HPLC and ion chromatography (IC) in order to measure the concentration profile of the components during the adsorption process. The concentrations of the aromatic acids found in the effluent and in the acidified aqueous solution before the treatment with Amberlite^{®} XAD-4 (Reference L/L R) are listed in Table 1 below and graphically represented in Figure 2.

**Table 1**

| Compound (mg/l) | Reference L/L R | 1 litre | 2.5 litres | 4.3 litres | 6.8 litres | 8.6 litres | 10.8 litres |
|---|---|---|---|---|---|---|---|
| Phthalic acid | 77 | 0.2 | 11 | 97 | 91 | 84 | 82 |
| 4-*tert*-amylphthalic acid | 361 | 0.1 | 0.5 | 1.3 | 5.7 | 13 | 69 |
| 4-*sec*-amylphthalic acid | 243 | < 0.1 | 0.2 | 0.5 | 2.1 | 4.1 | 26 |
| Amylphthalic acid derivatives | 139 | 0.7 | 5.4 | 31 | 115 | 173 | 197 |
| Other aromatic compounds | 150 | 2.9 | 16 | 50 | 79 | 92 | 106 |
| Total aromatic | 970 | 4.0 | 33.1 | 179.8 | 292.8 | 366.1 | 480.0 |

The results in Table 1 demonstrate the following:
The isomers of amylphthalic acid are most preferably adsorbed on the support. It is possible to treat up to 7 litres of effluent with 45 g of resin without releasing significant amounts of the amylphthalic acids (total concentration below 10 mg/l). This corresponds to an adsorption capacity of at least 90 g of amylphthalic acid by 1 kg of resin.

Phthalic acid is adsorbed in the beginning of the process then it is released by the column. The concentration gets to a plateau close to 80 mg/l which corresponds to the initial concentration in the effluent. After 4 litres of effluent, phthalic acid is not adsorbed by the column anymore.

Most of the unidentified compounds are not efficiently adsorbed by the column. These compounds correspond to more polar molecules coming from the oxidation of the amylphthalic acid isomers.

The compounds having the lowest affinity with the column are the ones included in the group of "derivatives of amylphthalic acid". Their concentration in the effluent after the column increases quickly after 4 litres of effluent treated.

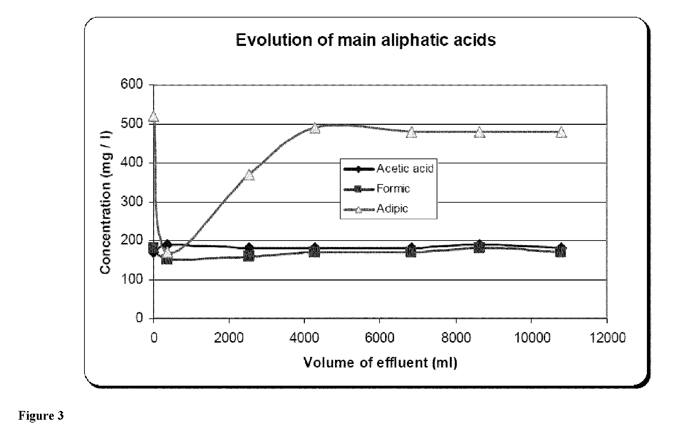

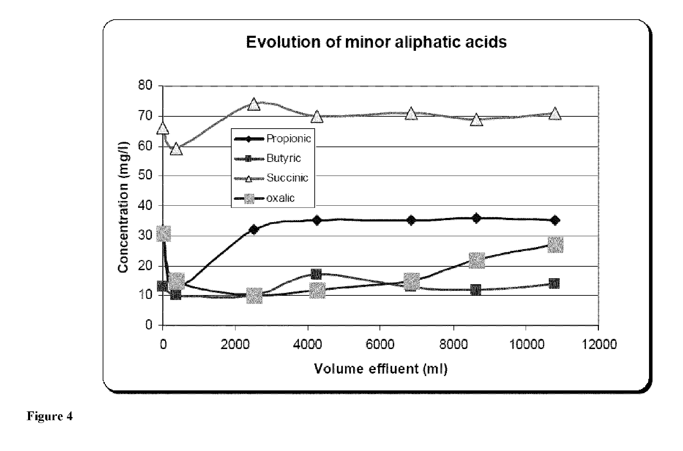

The results of the analysis of the aliphatic fraction in the effluent are summarized in Table 2 and illustrated by Figures 3 and 4.

**Table 2**

| Compound (mg/l) | ReferenceL/ L R | 1 litre | 2.5 litres | 4.3 litres | 6.8 litres | 8.6 litres | 10.8 litres |
|---|---|---|---|---|---|---|---|
| Lactic acid | <10 | <10 | <10 | <10 | <10 | <10 | <10 |
| Acetic acid | 170 | 190 | 180 | 180 | 180 | 190 | 180 |
| Propionic acid | 32 | 13 | 32 | 35 | 35 | 36 | 35 |
| Formic acid | 180 | 150 | 160 | 170 | 170 | 180 | 170 |
| Butyric acid | 13 | < 10 | < 10 | 17 | 13 | 12 | 14 |
| Adipic acid | 520 | 170 | 370 | 490 | 480 | 480 | 480 |
| Succinic acid | 66 | 59 | 74 | 70 | 71 | 69 | 71 |
| Oxalic acid | 31 | 15 | < 10 | 12 | 15 | 22 | 27 |

Acetic and formic acids are not absorbed by the column and their concentration in the effluent remains constant in time.

Adipic acid is adsorbed in the beginning of the process and up to an eluted volume of approximately 3 litres. This corresponds to a TOC value of 1000 mg/l.

This compound is progressively released and then it is not adsorbed anymore.

For the aliphatic acids present at a lower concentration level, some adsorption can be observed during the beginning of the treatment. This is especially true for propionic acid and, to a lesser extend, for succinic acid. These compounds are however released quickly form the column as the adsorption of aromatic compounds increases.

### 2. Comparative Example 1 - Tests performed at high pH

The following extraction tests were performed using a mixture of main stream water waste from a Solvay production unit. The mixture was used without any pretreatment and had a pH value of 13.

1 litre of aqueous solution was passed through the column containing 45 g Amberlite® XAD-4 and the effluent after the column was analyzed by HPLC.

The flow rate was 5 ml/min and the total residence time was 10 minutes.

The efficiency of the adsorption was rather limited since the TOC value after the column was close to 2000 mg/l starting from a value of 3250 mg/l for the aqueous solution before the treatment. Besides, the effluent after the column was not clear and slightly colored (yellow-orange).

The HPLC analysis of the aqueous solution before the column and of the effluent after the column is given in Table 3.

**Table 3**

| Compound (mg/l) | Before column | After column |
|---|---|---|
| Phthalic acid | 69 | 54 |
| 4-*tert*-Amylphthalic acid | 716 | 321 |
| 4-*sec*-Amylphthalic acid | 551 | 250 |
| Amylphthalic acid derivatives | 217 | 131 |
| Other aromatic compounds | 248 | 104 |
| Total aromatic | 1801 | 860 |

The efficiency of the adsorption was rather limited taking into account that the volume passed through the column was 1 litre.

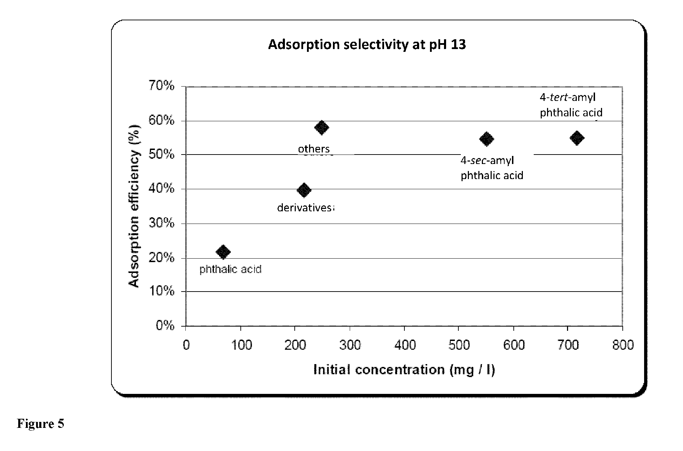

After saturation the column was washed with methanol to recover the adsorbed components. A selectivity analysis is given in Table 4 and is illustrated in Figure 5.

**Table 4**

| Compound (%) | Before column | After column | Methanol |
|---|---|---|---|
| Phthalic acid | 3.8 % | 6.3 % | 0.3 % |
| 4-*tert*-Amylphthalic | 39.8 % | 37.3 % | 43.5 % |
| 4-*sec*-Amylphthalic acid | 30.6 % | 29.1 % | 34.3 % |
| Amylphthalic acid | 12.0 % | 15.2 % | 6.2 % |
| Other aromatic | 13.8 % | 12.1 % | 15.7 % |
| Amylphthalic acid | 70.3 % | 66.4 % | 77.8 % |
| Ratio *(tert*/*sec)* | 57 / 43 | 56 / 44 | 56 / 44 |

There is a correlation between the initial concentration of the compound and its adsorption on the column. This means that the adsorption at pH 13 was not selective.

There is however, one point that is outside that correlation. The group of other aromatic compounds was adsorbed to higher extend than it should be according to their initial concentration (58 % instead of 50 %). This selectivity level is however, not sufficient for use as a fractionation tool.

### 3. Example 2 - Regeneration of the column

After having passed 10.8 litres of effluent on the column at pH 1.1 as described in Example 1, the column was regenerated using methanol as a solvent.

The amount of methanol used was 500 ml and the flow rate through the column was maintained at the same level (6 ml/min). Total residence time was 12 minutes.

A quick desorption leading to a yellow to orange methanol solution was observed. This solution was subsequently concentrated by evaporation of the methanol and a dark orange residue was obtained.

A sample of that residue was dissolved in alkaline water (1 N NaOH) and submitted to HPLC and IC analysis in order to determine its composition. The results are shown in Table 5.

**Table 5**

| Compound (mg/l) | Column desorption with methanol |
|---|---|
| Phthalic acid | 1.8 |
| 4-*tert*-Amylphthalic acid | 451 |
| 4-*sec*-Amylphthalic acid | 311 |
| Amylphthalic acid derivatives | 75 |
| Other aromatic compounds | 69 |
| Acetic acid | < 10 |
| Propionic acid | < 10 |
| Formic acid | < 10 |
| Butyric acid | < 10 |
| Adipic acid | < 10 |
| Succinic acid | < 10 |
| Oxalic acid | < 10 |

The concentrations of all aliphatic compounds were below the detection limit of 10 mg/l. The recovered fraction can therefore be considered as a fully aromatic.

Phthalic acid was present in a very small amount. This is consistent with the results described in Example 1 where it was observed that this compound was not retained on the column.

The composition of the aromatic fraction is presented in the following Table 6 and compared with the initial aromatic fraction.

**Table 6**

| Compound (%) | Reference L/L | Methanol extract |
|---|---|---|
| Phthalic acid | 7.9 % | 0.2 % |
| 4-*tert*-Amylphthalic acid | 37.2 % | 49.7 % |
| 4-*sec*-Amylphthalic acid | 25.1 % | 34.3 % |
| Amylphthalic acid | 14.3 % | 8.3 % |
| Other aromatic | 15.5 % | 7.6 % |
| Amylphthalic acid | 62.3 % | 83.9 % |
| Ratio *(tert*/*sec)* | 60 / 40 | 60 / 40 |

The recovered fraction contained mainly amylphthalic acid isomers with a total purity of 84 wt.-%. The isomeric ratio *tert*/*sec* was not affected by the purification process.

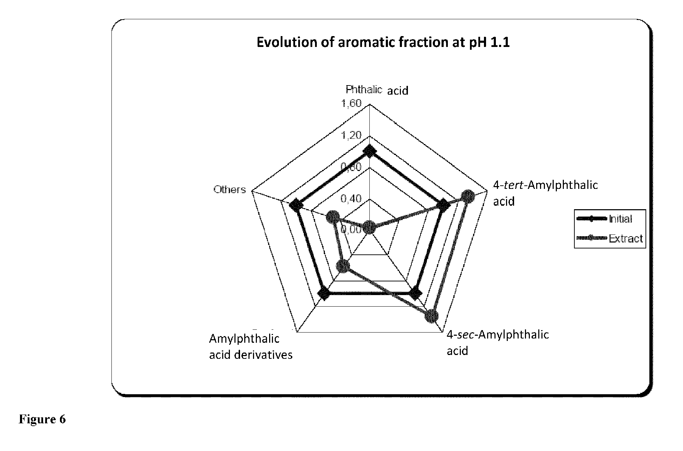

Figure 6 shows a normalized composition of the aromatic fraction before and after adsorption on Amberlite® XAD-4 at pH 1.1.

### 4. Example 3 - Column efficiency after regeneration

After methanol treatment described in Example 2, the column was washed with demineralized water. Then a volume of 2.5 litres of effluent used in Example 1 was passed through the column at the same flow rate as in the previous trials (6 ml/min).

The results are shown in Table 7.

**Table 7**

| Compound (mg/l) | Treatment of 2.5 litres on virgin resin | Treatment of 2.5 litres after resin regeneration |
|---|---|---|
| Phthalic acid | 11 | 15 |
| 4-*tert*-Amylphthalic acid | 0.5 | 0.9 |
| 4-*sec*-Amylphthalic acid | 0.2 | 0.4 |
| Amylphthalic acid | 5.4 | 6.5 |
| Other aromatic compounds | 16 | 17 |
| Acetic acid | 180 | 170 |
| Propionic acid | 32 | 31 |
| Formic acid | 160 | 180 |
| Butyric acid | < 10 | < 10 |
| Adipic acid | 370 | 400 |
| Succinic acid | 74 | 70 |
| Oxalic acid | < 10 | 27 |

Thus, after regeneration with methanol, the resin recovered its adsorption capacity. The concentration profiles of the aromatic and aliphatic compounds were similar to those obtained using virgin resin.

## Claims

1. A process for extraction of an aromatic dicarboxylic acid of Formula I 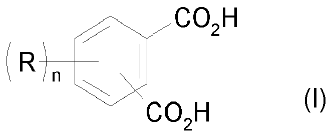 wherein
R is independently selected from the group consisting of a halogen atom, C₁₋₁₂alkyl and halogenated C₁₋₁₂alkyl,
n is 1, 2 or 3,
from an acidic aqueous solution, wherein the process comprises contacting the aqueous solution with a non-ionic polystyrene resin.

2. The process according to claim 1, wherein the non-ionic polystyrene resin is a para-divinylbenzene cross-linked polystyrene resin.

3. The process according to claims 1 or 2, wherein the specific surface area of the non-ionic polystyrene resin is higher than 700 m²/g.

4. The process according to any of claims 1-3, wherein the pore volume of the non-ionic polystyrene resin is higher than 0.45 ml/ml.

5. The process according to any of claims 1-4, wherein the non-ionic polystyrene resin is a *para*-divinylbenzene cross-linked polystyrene resin having
a specific surface area of not less than 750 m²/g,
a pore volume of not less than 0.5 ml/ml,
a particle size between 0.3 mm and 1.2 mm and
a pore envelope between 5.5 nm and 8.0 nm.

6. The process according to any of claims 1-5, wherein the acidic aqueous solution has a pH value below 3.0.

7. The process according to any of claim 1-6, wherein the acidic aqueous solution has a pH value below 1.5.

8. The process according to any of claims 1-7, wherein the aromatic dicarboxylic acid is a 4-alkylphthalic acid.

9. The process according to claim 8, wherein the 4-alkylphthalic acid is a 4-amylphtalic acid.

10. The process according to claim 9, wherein the 4-amylphthalic acid is selected from the group consisting of 4-*tert*-amylphthalic acid and 4*-sec-*amylphthalic acid.

11. A process for removal of the aromatic dicarboxylic acid as defined in claim 1 from an aqueous solution, wherein the process comprises the following steps:
a) adjusting the pH value of the aqueous solution to a value below 7.0;
b) extracting the composition obtained in step a) with a water non-miscible solvent;
c) extracting the aqueous solution obtained in step b) according to the process of any of claims 1-11; and
d) extracting the aromatic dicarboxylic acid from the non-ionic polystyrene resin with a water miscible solvent, wherein a solution of the aromatic dicarboxylic acid is obtained.

12. The process according to claim 11, wherein the water miscible solvent is selected from the group consisting of methanol, ethanol and acetone.

13. The process according to claims 11 or 12, wherein the solution of the aromatic dicarboxylic acid obtained in step d) is subsequently evaporated to yield the aromatic dicarboxylic acid having a purity above 80 wt.-%.

14. The process according to claims 11 or 12, wherein the aqueous solution obtained in step c) is subsequently subjected to biological treatment.

15. The process according to any of claims 11-14, wherein the aqueous solution is industrial waste water.
